# EUROPEAN PATENT SPECIFICATION

(11) **EP 4 229 640 B1**
(45) Date of publication and mention of the grant of the patent: **04.12.2024**
(21) Application number: 21791393.8
(22) Date of filing: 15.10.2021
(51) Int. Cl.: G16B 30/00, G16B 40/20

(54) **METHOD, SYSTEM AND COMPUTER PROGRAM PRODUCT FOR DETERMINING PEPTIDE IMMUNOGENICITY**
VERFAHREN, SYSTEM UND COMPUTERPROGRAMMPRODUKT ZUR BESTIMMUNG DER IMMUNOGENITÄT VON PEPTIDEN
PROCÉDÉ, SYSTÈME ET PRODUIT PROGRAMME D'ORDINATEUR POUR DÉTERMINER L'IMMUNOGÉNICITÉ D'UN PEPTIDE

(30) Priority: 15.10.2020 EP 20202140
(43) Date of publication of application: 23.08.2023
(73) Proprietor: MYNEO NV, 9000 Gent (BE)
(72) Inventor: PFITZER, Lena, 9000 Ghent (BE); FANT, Bruno, 9000 Ghent (BE); BOGAERT, Cedric, 9000 Ghent (BE)
(74) Representative: Brantsandpatents bv
(86) International application number: PCT/EP2021/078639
(87) International publication number: WO 2022/079255

(56) References cited:
- US-A1- 2013 330 335
- US-A1- 2016 132 631
- US-A1- 2019 346 442

## Description

### Fl ELD OF THE I NVENTI ON

The present invention pertains to a method, system and computer program product for determining peptide immunogenicity, as well as use thereof for cancer treatment, virus vaccination, etc.

### BACKGROUND

T-cell epitope prediction plays an important role in immunization experimental design and vaccine preparation.

Currently, most epitope prediction research focuses on peptide processing and presentation, e.g. proteasomal cleavage, transporter associated with antigen processing (TAP) and major histocompatibility complex (MHC) combination.

To date, however, the mechanism for immunogenicity of epitopes remains unclear. It is generally agreed upon that T-cell immunogenicity may be influenced by the foreignness, accessibility, molecular weight and structure, molecular conformation, chemical properties and physical properties of target peptides to different degrees.

For vaccine development (i.e. a cancer vaccine, virus vaccine), it is of the essence to identify peptides that can elicit an immune response. The potency of a vaccine is highly reliant on the immunogenicity of the epitopes, i.e. the ability of a possible target peptide to elicit an immune response. The current state-of-the-art for immunogenicity testing, involves assays that are labor- and time-intensive and require safety measures in a laboratory setting.

Current strategies for identifying peptides able to elicit an immune response use predicted binding affinity (IC50) to MHC molecules. Examples of such strategies are discussed in US 2013 330 335, US 2016 132 631 and US 2019 346 442. However, not all peptides with a high (predicted) binding affinity also elicit an immune response.

Using such binding prediction models which predict whether a peptide is likely to be presented on the MHC and assuming that those selected peptides are also immunogenic neoantigens, results in a large number of false positive predictions. As little as 5% of said neoantigens identified through such models for predicting peptide binding/presenting on the MHC are also able to elicit an immune response, highlighting the need for better identification of truly immunogenic peptides.

The IEDB immunogenicity predictor and Ineo-Epp are two publicly available immunogenicity prediction tools. The IEDB immunogenicity predictor is a simple model that rates peptides based on the position and properties of their amino acids. It is based on the position-wise enrichment of amino acids in immunogenic versus non-immunogenic peptides. The resulting immunogenicity score of a peptide is the sum of its individual amino acid scores. Ineo-Epp is a random forest classifier trained on HLA-I immunogenic peptides of which several characteristics, like amino acid physicochemical properties and eluted ligand likelihood percentile rank (EL rank(%)) score, were extracted. Ineo-Epp uses a list of peptides, together with an HLA allele as input. For antigen prediction, the nine main HLA-I supertypes (A1, A2, A3, A24, B7, B27, B44, B58, B62) can be used.

Both prediction tools, however, substantially underperform compared to the predictor system of the present invention. The present invention aims to provide improved immunogenicity prediction tools over the state-of-the-art techniques.

### SUMMARY OF THE I NVENTI ON

In a first aspect, the invention pertains to a computer-implemented method for determining immunogenicity of a peptide, according to claim 1.

In a second aspect, the invention pertains to a computer system for determining immunogenicity of a peptide, according to claim 11.

In a third aspect, the invention pertains to a computer program product for determining immunogenicity of a peptide, according to claim 12.

In a fourth aspect, the invention pertains to a use for determining a cancer treatment of a subject, particularly by determining immunogenicity of a neoantigens presented by a tumour cell of the subject, according to claim 13.

In a fifth aspect, the invention pertains to a use for screening of a virus or bacterium on a vaccination target, particularly by determining immunogenicity of epitopes from viral or bacterial proteins, according to claim 14.

In a sixth aspect, the invention pertains to a use for characterizing autoimmunity reactions of a subject, particularly by determining immunogenicity of self-antigens presented by a cell of the subject, according to claim 15.

In a seventh aspect, the invention pertains to a use for assessing immunogenicity changes of a peptide upon altering one or more amino acids of the amino acid sequence of said peptide, in particular simulating amino acid changes which can increase or decrease the immunogenicity of particular peptides, according to claim 16.

The present invention is advantageous as the machine learning classifier can be trained via sets of records, thereby learning to recognize immunogenicity of peptides based on an amino acid sequence thereof. This, for example, significantly simplifies the vaccine development process, as peptides can be selected based on a well-informed immunogenicity indication. Furthermore, benchmarking shows a significant improvement in the performance of the machine learning classifier of the present invention in comparison to other immunogenic peptide classifiers known in the art.

Preferred embodiments of the present invention are discussed in claims 2-10, as well as throughout the description, examples, and figures.

### DESCRI PTI ON OF FI GURES

**Figure** 1 shows a schematic workflow of the present invention.
**Figures 2 to** 5 show Receiver Operating Characteristic (ROC) and Precision-Recall benchmarking of different configurations and classifiers that are implemented according the present invention.
**Figure** 6 shows ROC and Precision-Recall benchmarking of the present invention to other immunogenic peptide classifiers known in the art.
**Figure 7** shows histogram and density curves of immunogenicity probability for the envelope-derived peptides of SARS-CoV-2, SARS-CoV and MERS-CoV.
**Figure 8** shows peptide sequence logos showing differential amino acid usage of 9 amino acid long peptides in the immunogenic positive dataset (above) over 9 amino acid long peptides in the non-immunogenic negative dataset (below). Amino acids were either ungrouped **(****Fig. 8A****),** grouped by chemical properties **(****Fig. 8B****),** electrical charge **(****Fig. 8C****)** or size **(****Fig. 8D****).**

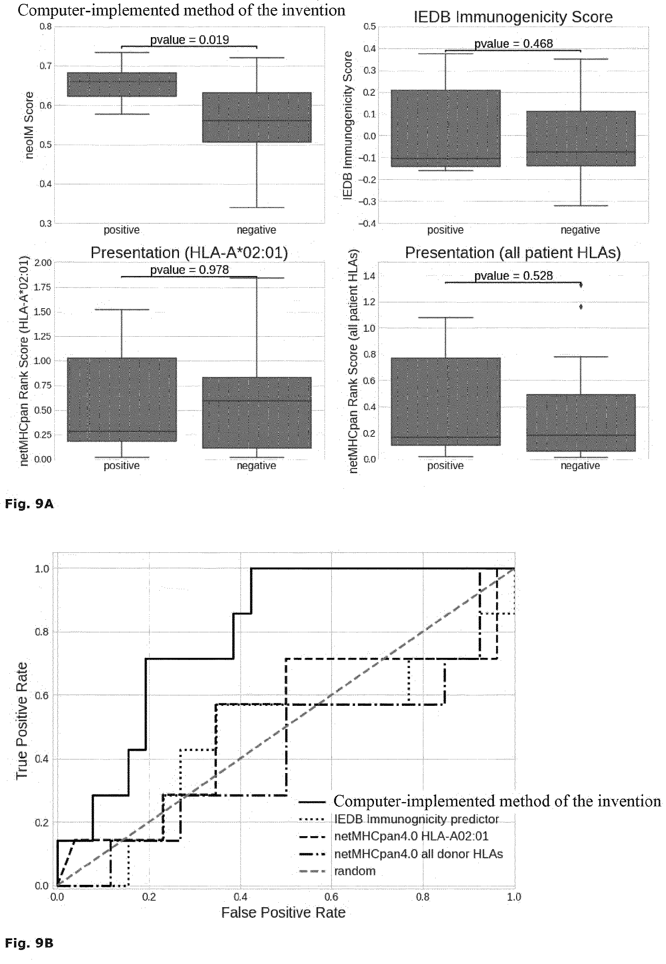

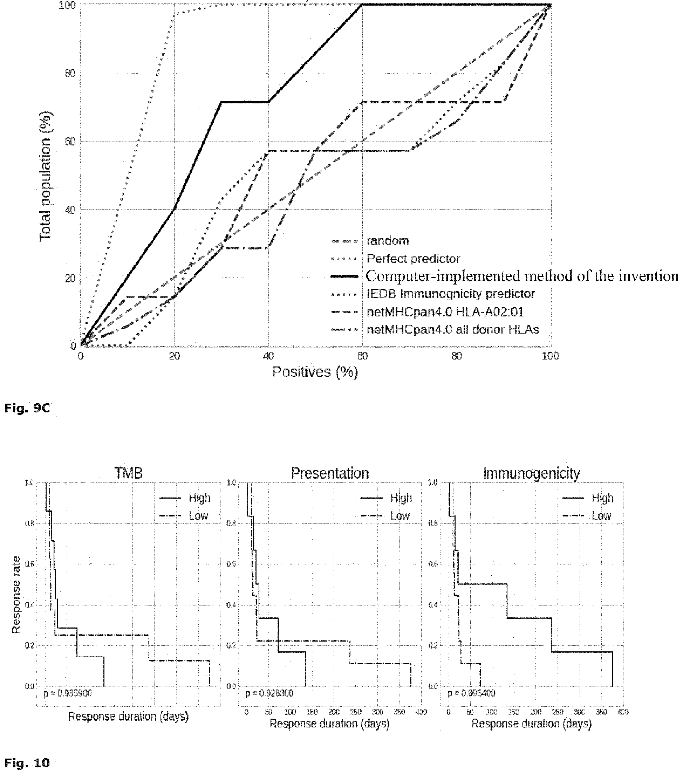

**Figure 9** shows the correlation of different parameters used to predict an immunogenic reaction with ELISpot results of 33 peptides which have all been predicted to be presented by netMHCpan4.0. **Fig. 9A** shows the predicted likelihood of peptides being immunogenic as assayed using the computer-implemented method of the invention, the IEDB immunogenicity predictor and netMHCpan4.0, the latter either based on a specific HLA allele or all HLA alleles of a subject, of pools of peptides with a positive or negative ELISpot result. **Fig. 9B** shows a comparison of receiver operating characteristics (ROC) curves of multiple different immunogenicity predictors, including the computer-implemented method of the invention, the IEDB immunogenicity predictor and netMHCpan4.0, the latter either based on a specific HLA allele or all HLA alleles of a subject. **Fig. 9C** shows the cumulative gains chart of multiple different immunogenicity predictors including the computer-implemented method of the invention, the IEDB immunogenicity predictor and netMHCpan4.0, the latter either based on a specific HLA allele or all HLA alleles of a subject. The cumulative gain curve illustrates what percentage of the true positives is identified with the smallest possible portion of samples.
**Figure 10** shows the response rate versus the response duration of melanoma patients to CTLA-4 blockade. Patients are divided into two groups, indicated as "High" (solid line) or "Low" (dotted line), based on the median number of mutations (tumor mutational burden TMB), the number of presented mutations or the number of immunogenic mutations.

### DETAI LED DESCRI PTI ON OF THE I NVENTI ON

The invention relates to a method, system and computer program product for determining peptide immunogenicity. Further, the invention relates to use of the method, the system and/or the product, for cancer treatment and virus vaccination. In what follows, the invention will be described in detail, preferred embodiments are discussed, and the invention will be illustrated by means of non-limitative examples.

Unless otherwise defined, all terms used in disclosing the invention, including technical and scientific terms, have the meaning as commonly understood by one of ordinary skill in the art to which this invention belongs. By means of guidance, term definitions are included to better appreciate the teaching of the present invention.

As used herein, the following terms have the following meanings:
"A," "an," and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include," "including," "includes" or "contain," "containing," "contains" and are inclusive or open-ended terms that specifies the presence of what follows e.g. component and do not exclude or preclude the presence of additional, non-recited element, members, steps, etc., known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Whereas the terms "one or more" or "at least one," such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

The recitation of numerical ranges by endpoints includes all numbers and fractions subsumed within that range, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the present invention pertains to a computer-implemented method for determining immunogenicity of a peptide according to claim 1.

In a second aspect, the present invention pertains to a computer system for determining immunogenicity of a peptide. The computer system being configured for performing the computer-implemented method according to the first aspect.

In a third aspect, the present invention pertains to a computer program product for determining immunogenicity of a peptide, wherein the computer program product comprises instructions which, when the computer program product is executed by a computer, cause the computer to carry out the computer-implemented method according to the first aspect.

In a fourth aspect, the present invention pertains to use of the computer-implemented method of the first aspect and/or the computer system of the second aspect and/or the computer program product of the third aspect, for determining a cancer treatment of a subject, particularly by determining immunogenicity of a neoantigens presented by a tumour cell of the subject.

In a fifth aspect, the present invention pertains to use of the computer-implemented method of the first aspect and/or the system of the second aspect and/or the product of the third aspect, for screening of a virus on a vaccination target, particularly by determining immunogenicity of epitopes from viral proteins.

In a sixth aspect, the invention pertains to a use of the computer-implemented method of the first aspect and/or the system of the second aspect and/or the product of the third aspect, for characterizing autoimmunity reactions of a subject, particularly by determining immunogenicity of self-antigens presented by a cell of the subject.

In a seventh aspect, the invention pertains to a use of the computer-implemented method of the first aspect and/or the system of the second aspect and/or the product of the third aspect, for screening of a bacterium on a vaccination target, particularly by determining immunogenicity of epitopes from bacterial proteins.

In an eight aspect, the invention pertains to a use of the computer-implemented method of the first aspect and/or the system of the second aspect and/or the product of the third aspect, for assessing immunogenicity changes of a peptide upon altering one or more amino acids of the amino acid sequence of said peptide. In particular, a use for simulating amino acid changes which can increase or decrease the immunogenicity of particular peptides.

The invention provides a computer-implemented method, a computer system and a computer program product for determining immunogenicity of a peptide, as well a use of any of any of the method, system or product for cancer treatment, virus vaccination, characterizing autoimmunity reactions of a subject, screening of a bacterium or other pathogens on a vaccination target and for assessing immunogenicity changes of a peptide upon altering the amino acid sequence of said peptide. A person having ordinary skill in the art will appreciate that the method is implemented in the computer program product and executed using the computer system. It is also clear for a person having ordinary skill in the art that immunogenicity prediction can be used for cancer treatment, virus vaccination, characterizing autoimmunity reactions of a subject and screening of a bacterium on a vaccination target. Therefore, all of the eight aspects of the present invention are hereafter treated together.

A simple embodiment of the invention preferably provides obtaining an amino acid sequence of the peptide wherefore immunogenicity is to be determined. Preferably, the peptide is a cell-surface presented peptide. More preferably, a cell-surface presented major histocompatibility complex (MHC) bound peptide. Said peptides preferably either bind MHC class I and trigger a CD8 T cell response, or bind MHC class II and trigger a CD4 T cell response. The invention is therefore applicable in some embodiments to MHC I alleles and in some embodiments to MHC II alleles. Preferably, peptide sequences for which immunogenicity will be predicted using the invention (query sequences) should be known to be presented by MHC. The advantage thereof is that the said query peptides will more closely resemble the peptides from which the training dataset (see below) is derived and that are all known to be presented on MHC. Therefore, properties relating to immunogenicity and not MHC presentation are the most dominant distinguishing factors between positive and negative peptides (see below).

The peptide can be obtained from any subject. "Subject," as used herein, refers to a term known in the state of the art, that should preferably be understood as a human or animal body, most preferably a human body. As used herein, "animal" preferably refers to vertebrates, preferably to birds and mammals, even more preferably mammals. "Subject in need thereof," as used herein, should be understood as a subject who will benefit from (prophylactic) treatment, e.g. cancer or virus vaccination. Furthermore, the peptide wherefore immunogenicity is to be determined comprises a sequence of amino acids (aa) and preferably has a length between 9 and 11 aa, or a length between 8 and 12, or a length between 13 and 25.

A simple embodiment of the invention preferably provides obtaining for each proteinogenic amino acid a plurality of numeric indices, wherein each of said numeric indices are related to a physicochemical property of a corresponding proteinogenic amino acid. "Proteinogenic amino acids," as used herein, are amino acids that are incorporated biosynthetically into proteins during translation. "Proteinogenic" means "protein creating". Throughout known life, there are 22 genetically encoded (proteinogenic) amino acids, 20 in the standard genetic code and an additional 2 that can be incorporated by special translation mechanisms. In contrast, "non-proteinogenic amino acids" are amino acids that are either not incorporated into proteins (like GABA, L-DOPA, or triiodothyronine), disincorporated in place of a genetically encoded amino acid, or not produced directly and in isolation by standard cellular machinery (like hydroxyproline). The latter often results from post-translational modification of proteins. By obtaining information on proteinogenic acids, information on the "building-blocks" of each peptide of a subject is obtained. A peptide may thus comprise proteinogenic amino acids, non-proteinogenic amino acids, or a combination of both.

The physicochemical properties are preferably obtained from a database known in the art, such as the AAindex database at 'https://www.genome.jp/aaindex/'. The AAindex is a database of numerical indices representing various physicochemical and biochemical properties of amino acids and pairs of amino acids. AAindex consists of three sections now. The section of interest is the AAindex1 comprising the amino acid index of 20 numerical values. Examples of physicochemical properties include, but are in no way limited to, hydrophobicity, free energy, aa distribution, etc.

A simple embodiment of the invention preferably provides training a classification model on the training data set, and determining the likelihood of said peptide eliciting an immune response by means of the trained classification model. In the context of training of mathematical models such as classification algorithms, following terms are used, and hereby by means of guidance further explained.

The "training set" is the set of data observations (also called 'records,' 'examples,' or 'instances') that is used to train or to learn the model. An analytical model has parameters that need to be estimated in order to make good predictions. This translates into finding the optimal parameter values for the analytical model. For this reason, we use the training set to find or to estimate the optimal parameter values. Once we have a trained model, we can use it to make predictions. In a supervised classification task, also class labels (e.g., 'immunogenic', 'non-immunogenic') are attached to each observation to estimate the optimal parameter values. This allows to train the algorithm on patterns that are helpful to identify fraud cases.

The "validation set" relates to models with parameters that cannot be estimated directly from the data. Yet, in order to also find optimal values for those parameters (referred to as hyperparameters), the so-called validation set is used. Typically, a set of candidate values for the hyperparameters can be identified. One picks one candidate value, trains the model on the training set, and evaluates the prediction performance on the validation set. Then one picks the next candidate value and proceeds in a similar fashion until all candidate values have been tried out. In the end, for each candidate value a corresponding estimate of the prediction performance is obtained. Based on the performances estimated on the validation set, one can pick the one candidate value that corresponds to the optimal performance. The training set and validation set are preferably strictly separated in the whole process in order to obtain reliable performance estimates. That is, observations in the validation set cannot be in the training set (or test set for that matter). Alternatively, the training set and the validation set are not strictly separated. This is for example the case with cross-validation.

The "test set," also "hold-out sample," is the set of data observations that is used to test whether the trained model makes good predictions. That is, in the model evaluation phase, one knows the true values of the test observations and one may check how many of the predicted values are correct by comparing them to the true values. It is important to note that here the class labels are only used to evaluate the prediction performance (e.g., accuracy) of the classification model. That is, observations in the test set cannot be in the training set or validation set. The strict separation is crucial, because one desired the model to make prediction about observations that have not been used in the training process. Only when this is guaranteed and the model shows a good performance, one can be certain that the model will also perform well on new, previously unseen data.

The "holdout strategy" or "single train-test split strategy" refers to the simplest splitting because the data is divided into two sub-sets: one for training and one for testing. One may train the model with the former and then test it with the latter. Note that the train-test process is only done once. This data split is done randomly, i.e. observations are randomly assigned to belong to the training or test set. The performance is evaluated on the test set, usually for a set of candidate models, and the best model is picked. Furthermore, it is often necessary to compare trained and validated model, in order to take into account overfitting. Some models possess parameters that cannot be estimated from the data directly. They are called hyperparameters. One may rely on a validation set to find the best model. Here, one can divide the data into three subsets: one for training, one for validation, and one for testing. The splitting is also done in a random fashion. With the help of the validation set, one can find the model with the optimal hyperparameter values (i.e. a model selection), and the best model is finally evaluated on the test set. Note that the choice for the selection of the best prediction model, amongst a set of various candidate models, is made based on the performance measured on the test set, e.g. one may need to decide if the logistic regression model, decision tree, or random forest is the best performing model. To make this decision, performance on the test set is crucial. When the final prediction model is found, it may be put it into practice in the operational system for making predictions for new, previously unseen data.

The term "k-fold cross validation strategy" refers to an alternative to the simple train-test splitting. It corresponds to a repetitive train-test splitting, whereby the test set is shifted systematically. The obtained performances on test sets are then averaged. The advantage of this strategy is that each observation will be once in the test set. Yet, more importantly, the estimated prediction performance becomes more reliable, which provides a better picture of the generalization performance of the model.

The training data set of the present invention at least comprises a positive data set that comprises a plurality of amino acid sequences of immunogenic peptides. "Immunogenicity" or "immunoreactivity," as used herein, results from a biomaterial being detected by the body's immune system as a foreign object. Immunoreactive biomaterials such as peptides are detected by antigenic reactions on cells. A biochemical cascade then occurs, whereby T-helper cells migrate towards the biomaterial. Accordingly, the classification model is trained to recognize immunogenicity.

According to a preferred embodiment, the immunogenic peptides of the positive data set are obtained by: obtaining amino acid sequences of peptides capable of inducing a T-cell response; obtaining amino acid sequences corresponding to proteome peptides; comparing the T-cell response inducing amino acid sequences to the proteome amino acid sequences to determine matches therebetween; the positive data set comprising the T-cell response inducing amino acid sequences except for the amino acid sequence of said matches. Preferably, the obtained T-cell response inducing amino acid sequences are linear sequences.

According to an embodiment for determining autoimmune peptide immunogenicity, the immunogenic peptides of the positive data set are obtained by: obtaining amino acid sequences of peptides capable of inducing a T-cell response; obtaining amino acid sequences corresponding to proteome peptides; comparing the T-cell response inducing amino acid sequences to the proteome amino acid sequences to determine matches therebetween; the positive data set comprising the T-cell response inducing amino acid sequences of said matches. Preferably, the obtained T-cell response inducing amino acid sequences are linear sequences.

According to an embodiment for determining the immunogenicity of peptides resulting from mutations, the immunogenic peptides of the positive data set are obtained by: obtaining amino acid sequences of peptides capable of inducing a T-cell response; obtaining amino acid sequences corresponding to proteome peptides; comparing the T-cell response inducing amino acid sequences to the proteome amino acid sequences to determine matches therebetween; the positive data set comprising the T-cell response inducing amino acid sequences that are closely related to but not identical to peptides of the (e.g. human)proteome. Preferably, "closely related to but not identical to" means that 1, 2 or 3 amino acid mismatches in between said peptides are allowed. A person skilled in the art will know methods to compare query peptides (peptides to be assessed) with peptides of the human proteome and how to identify the number of mismatches. The advantage thereof is that by filtering out peptides that are identical to peptides of the human proteome, peptides in the positive dataset resemble more closely the peptides of interest which are peptides that have an altered sequence due to mutation.

According to an embodiment, the immunogenic peptides of the positive data set are obtained by: obtaining amino acid sequences of peptides capable of inducing a T-cell response; obtaining amino acid sequences corresponding to proteome peptides; comparing the T-cell response inducing amino acid sequences to the proteome amino acid sequences to determine matches therebetween; the positive data set comprising the T-cell response inducing amino acid sequences having 1 or more, 2 or more, 3 or more, 4 or more, 5 or more, ... mismatches with peptides of the (e.g. human) proteome. A person skilled in the art will know methods to compare query peptides (peptides to be assessed) to peptides of the (human) proteome and how to identify the number of mismatches. This positive data set may be used when a model is required which is specific for foreign (e.g. - if compared to human proteome sequences - non-human or non-mammalian e.g. bacterial or viral) antigens. Said positive data set obtained in relation to peptides having multiple mismatches to peptides of the (human) proteome may resemble a positive data set obtained from said foreign (e.g. bacterial or viral) antigens.

The peptides capable of inducing T-cell response can be experimentally characterized by a human T-cell assay. The T-cell assay can measure cytokine release, cytotoxicity, or qualitative T-cell binding to an antigen presenting cell (APC). The cytokine measured for a T-cell assay can be selected from the group consisting of interferon gamma (IFNy), tumor necrosis factor alpha (TNFa), interleukin-2 (IL-2), interleukin-4 (IL-4), interleukin-5 (IL-5), interleukin-6 (IL-6), interleukin-8 (IL-8), interleukin- 10 (IL-10), interleukin- 17 (IL-17), interleukin-21 (IL-21), interleukin-22 (IL-22), granzyme A, and granzyme B. In some variations, the qualitative T-cell binding can be determined by MHC multimer staining. In some variation, the T-cell assay can be performed ex vivo or in vitro. The experimentally characterized peptides recognized by T-cells can be selected from the Immune Epitope Database (IEDB).

According to a preferred embodiment, the training data set further comprises a negative data set.

Said negative data set may be obtained by: obtaining amino acid sequences of peptides capable of binding to and/or being presented on the major histocompatibility complex (MHC), preferably identified by means such as binding assays and/or mass spectrometry data; obtaining amino acid sequences corresponding to housekeeping proteome peptides; comparing said MHC presented/MHC binding amino acid sequences to the housekeeping proteome amino acid sequences to determine matches therebetween; the negative data set comprising the matches therebetween i.e. the MHC presented amino acid sequences that are corresponding to housekeeping proteins. Said binding assays and/or mass spectrometry for identifying peptides capable of binding to and/or being presented on the MHC may be any type of binding assays and/or mass spectrometry known in the art. Types of binding assays and/or mass spectrometry are known by a person skilled in the art. Preferably, the obtained MHC presented amino acid sequences are linear sequences.

In the present context, "housekeeping proteome" refers to a set of proteins involved in the basic functions of a cell or set of cells in an organism.

According to an embodiment for determining the immunogenicity of peptides resulting from mutations, the negative data set is obtained by: obtaining amino acid sequences of peptides capable of binding and/or being presented on the major histocompatibility complex; obtaining amino acid sequences corresponding to proteome peptides; comparing said MHC presented/MHC binding amino acid sequences to the proteome amino acid sequences to determine matches therebetween; the negative data set comprising the MHC presented/MHC binding amino acid sequences that are closely related to but not identical to peptides of the human proteome. Preferably, "closely related to but not identical to" means that 1, 2 or 3 amino acid mismatches in between said peptides are allowed. A person skilled in the art will know methods to compare query peptides (peptides to be assessed) with peptides of the (e.g. human) proteome and how to identify the number of mismatches. The advantage thereof is that by filtering out peptides that are identical to peptides of the (human) proteome, peptides in the negative dataset resemble more closely peptides that have an altered sequence due to mutation. By choosing this negative dataset it is avoided that the classification model would be trained to distinguish mutated peptides from non-mutated peptides instead of immunogenic from non-immunogenic peptides. Another or linked advantage is that the negative data set will closely resemble the peptides that will be tested by the model, thereby increasing the specificity of the model.

According to an embodiment, the negative data set is obtained by: obtaining amino acid sequences of peptides capable of being presented the major histocompatibility complex; obtaining amino acid sequences corresponding to proteome peptides; comparing said MHC presented amino acid sequences to the proteome amino acid sequences to determine matches therebetween; the negative data set comprising the MHC presented amino acid sequences that are having 1 or more, 2 or more, 3 or more, 4 or more, 5 or more, ... mismatches with peptides of the (e.g. human) proteome. A person skilled in the art will know methods to compare query peptides (peptides to be assessed) to peptides of the (human) proteome and how to identify the number of mismatches. This negative data set may be used when a model is required which is specific for foreign (non-human or non-mammalian e.g. bacterial or viral) antigens. Said negative data set obtained from peptides having multiple mismatches to peptides of the human proteome may resemble a negative data set obtained from said foreign (e.g. - if compared to human proteome sequences - non-human or non-mammalian e.g. bacterial or viral) antigens.

According to a preferred embodiment, the training data set comprises a positive and negative data set, wherein the negative data set comprises substantially more records compared to the positive data set. Preferably, at a ratio of at least 3:1. According to another embodiment, the training data set comprises a positive and negative data set, wherein the negative data set comprises a similar amount of records compared to the positive data set, or less records compared to the positive data set.

According to a preferred embodiment, the classification model is a classification machine learning algorithm, preferably a supervised classification machine learning algorithm, more preferably wherein the machine learning algorithm is classification algorithm is one or more of a Multilayer Perceptron classifier, a Decision Tree Classifier, a Gaussian Naive Bayes classifier, a Gaussian Process classifier, a Stochastic Gradient Descent classifier, a Linear Support Vector Machine, a Kernel Support Vector Machine, a K-Nearest Neighbour classifier, or a Random Forest classifier. After benchmarking the Random Forest classifier shows the best results. Moreover, the inventors note that Random Forest classifiers algorithms require the least amount of work in terms of feature engineering and parameter tuning. Accordingly, the most preferred classification model according to the present invention is a Random Forest classifier.

A simple embodiment of the invention preferably provides, prior to training of the classification model, performing a principal component analysis on the numeric indices of each of the proteinogenic amino acids to obtain several principal components for each analysed proteinogenic amino acid. A "principal component analysis" or short "PCA," as used herein, is employed to reconstruct the most discriminative feature subspaces, which are subsequently used as input in representation-based classification for prediction. PCA improves prediction as well as processability of the data, but furthermore removes noise if restricted to a limited number of principal components. Transformation of amino acid properties in principal components reduces dimensionality and sets the focus on the most distinctive properties between the amino acids.

A simple embodiment of the invention preferably provides, prior to training of the classification model, obtaining a feature vector for the amino acid sequences of the training data set and the amino acid sequence of said peptide, wherein a feature vector for an amino acid sequence is obtained by replacing each amino acid of said amino acid sequence by one or more main corresponding principal components; wherein the classification model is trained on the feature vectors of the training data set; and wherein the likelihood of said peptide being immunogenic is determined for the feature vector of said peptide. Translating the peptides into feature vectors preserves positional information of the physicochemical properties.

Preferably, one or more main principal components, is to be understood as one or more of the first principal components, more preferably the 10 first principal components, even more preferably the 9 first principal components, even more preferably the 8 first principal components, even more preferably the 7 first principal components, even more preferably the 6 first principal components, even more preferably the 5 first principal components, even more preferably the 4 first principal components, and most preferably the 3 first principal components.

Accordingly, a feature vector for an amino acid sequence is obtained by replacing each amino acid of said amino acid sequence by between 2 and 10 corresponding main principal components, as well as any range therebetween. Most preferably a feature vector for an amino acid sequence is obtained by replacing each amino acid of said amino acid sequence by 3 corresponding main principal components. The inventors note that in their experience, restricting the feature vector to only the first three principal components prevent excessive processing requirements while retaining enough information for a very accurate prediction.

Additionally, prior to the principal component analysis, the plurality numeric indices are preferably transformed to z-values, which characterizes the probability that each feature will conform to a normal value.

According to an embodiment, the invention further provides training an unsupervised training algorithm for further defining characteristics of immunogenic and/or non-immunogenic peptides.

According to an embodiment, the invention further provides obtaining properties on a peptide level, such as peptide solubility, molecular weight, peptide stability, peptide 3-dimensional structure, global location of the peptide in the genome (e.g. coded as distance to the nuclear centre), similarity to non-mutated peptide, etc., which can be used to further train the classification model.

As will be clear to a person skilled in the art, the computer-implemented method, computer system and the computer program product of the present invention may be used to identify immunogenic peptides (immunogenic epitopes) within a set of MHC presented peptides (epitopes) and lead to the identification of therapeutic targets. Additionally, it can be used to assess immunogenicity changes of a peptide upon altering one or more amino acids of the amino acid sequence of said peptide, in particular to simulate amino acid changes which can increase or decrease the immunogenicity of particular peptides.

### EXAMPLES AND DESCRI PTI ON OF FI GURES

The invention is further described by the following non-limiting examples which further illustrate the invention, and are not intended to, nor should they be interpreted to, limit the scope of the invention.

### Example 1: Preferred Embodiment

The present example pertains to preferred embodiment of predicting the likelihood of a cell surface-presented peptide to elicit an immune response.

This present example accepts input peptides of 9-11 amino acid length that are presented on a cell surface (determined by means of a prediction algorithm or experimentally).

The output of the present example is a probability score between 0 and 1, describing the likelihood of the peptide being immunogenic.

### Pre-Processing

The inputs are pre-processed as follows:
Physicochemical properties for the 20 proteinogenic amino acids were downloaded from the AAindex database at 'https://www.genome.jp/aaindex/' (dated 13.2.2017). The AAindex is a database of numerical indices representing various physicochemical and biochemical properties of amino acids and pairs of amino acids. AAindex consists of three sections now: AAindex1 for the amino acid index of 20 numerical values, AAindex2 for the amino acid mutation matrix and AAindex3 for the statistical protein contact potentials. All data is derived from published literature. Numerical values from AAindex1 were transformed to z-values (mean set to 0 and standard deviation of 1). A principal component analysis was performed on the scaled physicochemical properties using the sklearn PCA module to reduce the dimensions of the data. Each amino acid in a peptide is then translated into the values of the 3 first principal components of the respective peptide, resulting in a feature vector of length (9-11)*3. If the length of the peptide is shorter than 11 amino acids, the missing amino acids are coded in as three zeros each at the N-terminus (peptide start). Therefore, the feature vector is always of length 11*3.

### Supervised Classifier:

The present example uses a Random Forest classifier module of the sklearn python package, particularly:
RandomForestClassifier(n _estimators= 1000,criterion='entropy',max_depth = None, min_samples_split=3,min_samples_leaf=1,max_features=2,bootstrap=False).

### Training Classifier

The training dataset includes peptides of length 9, 10 and 11 amino acids and 3-fold more negative than positive peptides. The training dataset is transformed into a matrix. Each peptide in the dataset was translated into a feature vector as described above. Each row in the matrix therefore corresponds to the feature vector of one of the peptides in the dataset. This matrix was used to train the random forest model described above and the trained model was saved.

### Training Data

The training data for training the supervised classifier is constructed as follows:

### Negative data (non-immunogenic peptides):

Peptides from endogenous housekeeping genes are assumed non-immunogenic as they are constantly presented on all healthy cells. Therefore, a negative training dataset is built from peptides originating from said housekeeping genes that have been shown to be presented on MHC molecules. MHC presentation prediction is not the goal of the present model. Moreover, we do not want it to be a factor in the model. Positive peptides as well as negative peptides are thus MHC binders. IEDB data of MHC binding peptides was imported from 'https://www.iedb.org/database_ export_v3.php' (version Feb. 19, 2020). Peptides were also filtered to be a linear sequence, from homo sapiens, result in at least one positive assay and be of length 9, 10 or 11 amino acids. The resulting peptide list contains only unique peptides.

Furthermore, housekeeping genes were retrieved from 'https://www.proteinatlas .org/human-proteome/tissue/housekeeping'. These were filtered to exclude genes annotated with 'Disease involvement' or 'RNA cancer specificity'. Fasta sequences of housekeeping genes proteins were retrieved from Uniprot based on the gene IDs from the previous step and a blast database was built from those sequences. The IEDB MHC binding peptides were then queried against the blast database of housekeeping genes. All the perfect matches of peptides (peptides resulting from these housekeeping genes) were retained as negative dataset.

### Positive dataset (immunogenic peptides):

The positive data (i.e. immunogenic peptides) is obtained from the IEDB database (dated Feb. 19, 2020): peptides with associated T-cell assay data. The peptides were furthermore filtered to be a linear sequence, tested for homo sapiens, resulting in at least one positive assay and be of length 9 amino acids. The resulting peptide list contains only unique peptides. After filtering the peptides, it is made sure that there is no overlap with the human proteome by filtering out perfect matches to human proteins. This is achieved by filtering out peptides with perfect matches to blast database made from the human proteome.

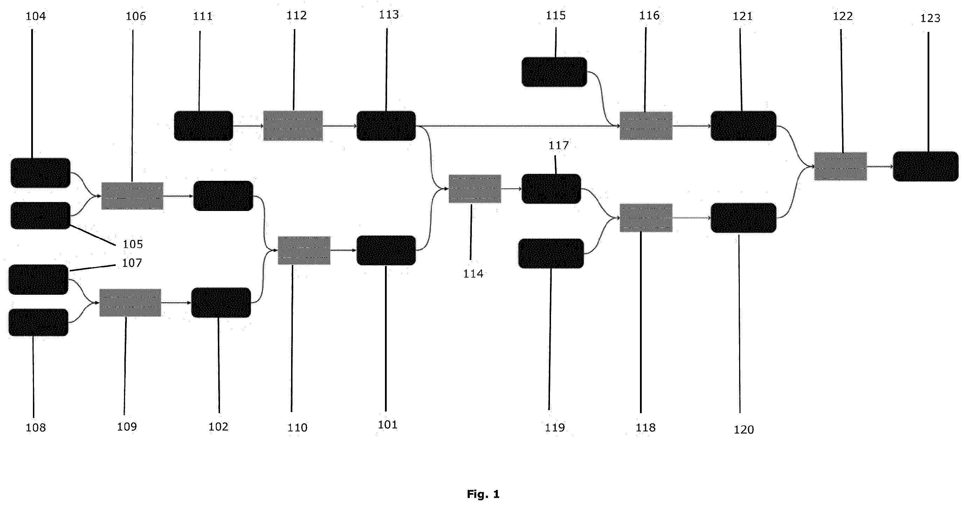

**Figure 1** illustrates the workflow of the present example as discussed hereabove. The training dataset (1) comprises both a positive (102) and negative data set (103). The negative data set is obtained by comparing the MHC presented amino acid sequences/sequences of peptides capable of binding to and/or being presented on MHC (104) to the housekeeping amino acid sequences (105) to determine matches (106) therebetween, wherein the negative data set comprises the amino acid sequences of said matches. The positive data set is obtained by comparing the T-cell response inducing amino acid sequences (107) to the proteome amino acid sequences (108) to determine matches (109) therebetween, wherein the positive data set comprising the T-cell response inducing amino acid sequences except for the amino acid sequence of said matches. Both negative and positive data set are 3:1 compiled (110) in the training data set.

Figure 1 furthermore shows the pre-processing according to the present example. Firstly, a plurality of numeric indices (111), each related to a physicochemical property, are obtained for each proteinogenic amino acid. Secondly, for each of the proteinogenic amino acids, a principal component analysis (112) is performed on said numeric indices. Thus, the principal numeric components (113) are determined for each of the proteinogenic amino acids. These can subsequently be used for a peptide to feature translation of both the peptides in the training data set (114), as well as for the peptide of interest (115, 116). The resulting feature matrix (117) is used for training (118) the Random Forest classifier (119) to obtain a trained classifier (120), which can be used to predict (122) immunogenicity for the peptide of interest by means of the therefore determined feature vector (121). The prediction obtained according to the present example is preferably shown as a score (123).

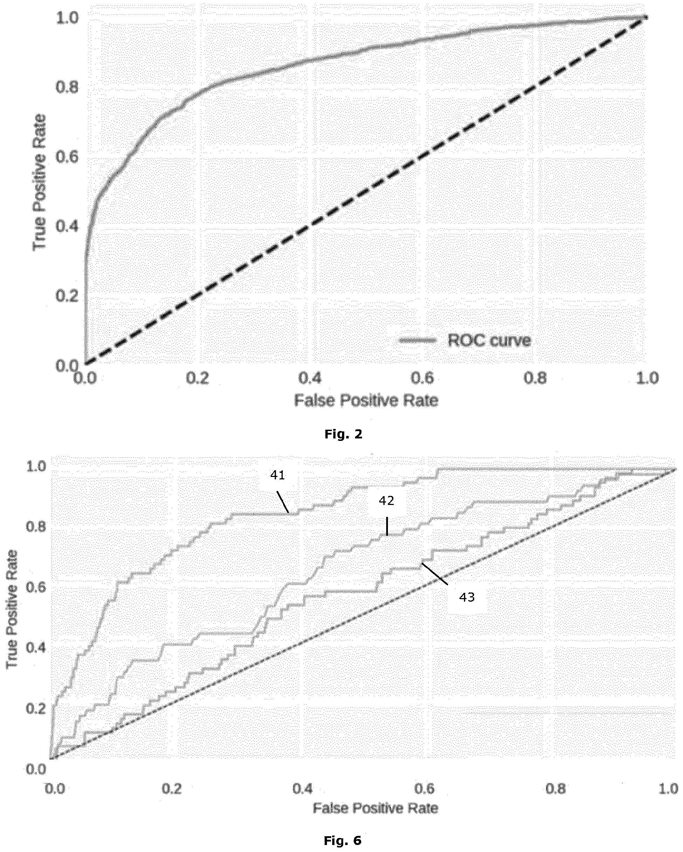

**Figure 2** shows the receiver operating characteristic (ROC) of the present example. Accordingly, the true positive rate (proportion of actual immunogenic epitopes that were called as such, i.e. sensitivity) is shown in function of the false positive rate (proportion of non-immunogenic epitopes called as immunogenic, i.e. 1- specificity). The precision-recall curve shows the trade-off between precision and recall for different thresholds. The Area Under Curve (AUC) surface of the ROC curve is 0.88.

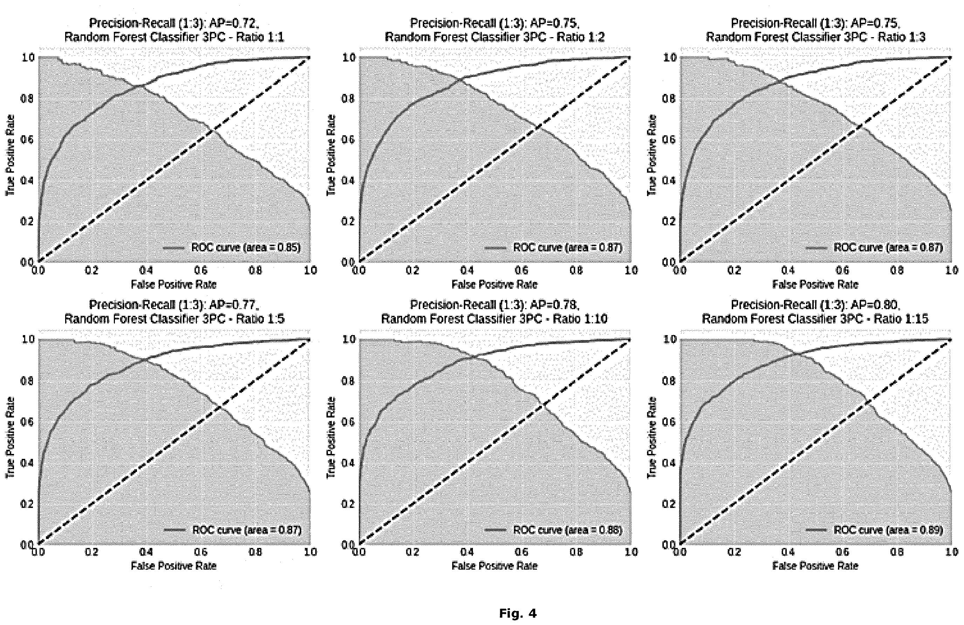

**Figure 4** shows ROC results of training with different ratios of positive to negative data, while validating on a 1:3 ratio. By increasing the ratio of negative data to positive data, results are improved.

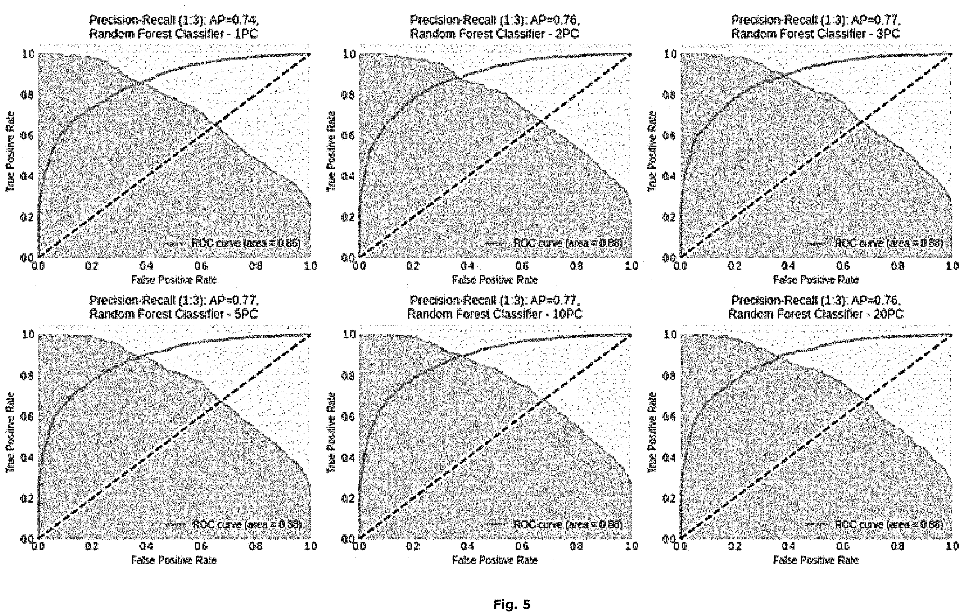

**Figure 5** shows metrics for training and validation using 1,2,3,5,10 or 20 of the principal components, respectively. Performance is increased when using 2 or more principal components. However, when using more than 10 principal components processing significantly increases.

### Example 2: Benchmark Classifiers

The present example pertains to performance of supervised classification algorithms available on scikit learn.

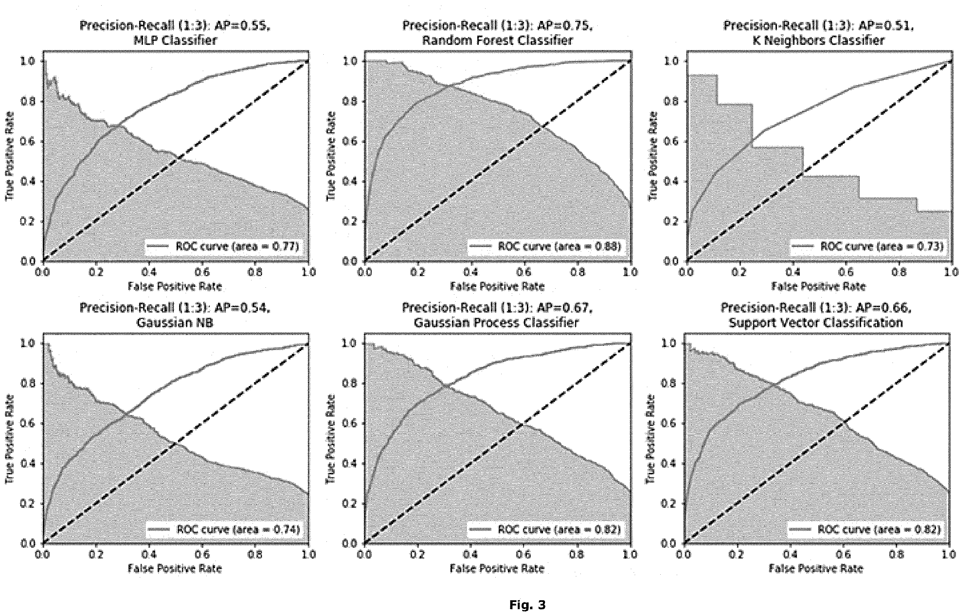

**Figure 3** shows Receiver Operating Characteristic (ROC) and Precision-Recall benchmarking of different classifiers known in the art. The workflow characteristics of Example 1 are adhered to for the implementation of each of the classifiers.

The tested classification algorithms include: a Multilayer Perceptron (MLP) classifier (Figure 3; 1*1), a Random Forest classifier (Figure 3; 1*2), a K-Nearest Neighbour classifier (Figure 3; 1*3), a Gaussian Naive Bayes classifier (Figure 3; 2*1), a Gaussian process classifier (Figure 3; 2*2), a Support Vector classifier (Figure 3; 2*3). The corresponding AUC values are: 0.77; 0.88; 0.73; 0.74; 0.82; 0.82.

Although all of the classifiers are effective with the current workflow, the Random Forest classifier clearly outperforms the other tested classifiers.

### Example 3: Benchmark Prior-Art

The present example pertains to a benchmarking of the workflow of Example 1 to other immunogenic peptide classifiers known in the art.

The workflow according to Example 1 was compared in a benchmark study with two other publicly available immunogenicity prediction tools, particularly the IEDB immunogenicity predictor and Ineo-Epp.

The IEDB immunogenicity predictor is a model that rates peptides based on the position and properties of their amino acids. Ineo-Epp is a random forest classifier trained on HLA-I immunogenic peptides of which several characteristics, like amino acid physicochemical properties and eluted ligand likelihood percentile rank (EL rank(%)) score, were extracted. Ineo-Epp uses a list of peptides, together with an HLA allele as input. For antigen prediction, the nine main HLA-I supertypes (A1, A2, A3, A24, B7, B27, B44, B58, B62) can be used.

For this benchmark study, immunogenic peptides were retrieved from the immune epitope database (IEDB) (accessed 15.06.2020). To avoid a bias in the benchmark data for epitopes present in the training data of any of the tools, antigen epitope data was retrieved from recent publications of 2020. The data was further filtered (Linear epitope, Human, positive T-cell assays) and cleaned from epitopes that were already deposited in earlier versions of the database. This reduced the number of positive peptides from 2213 to 64 unique epitopes, all of length 9 amino acids. As negative data, non-immunogenic peptides published in 'Chowell, D. et al. TCR contact residue hydrophobicity is a hallmark of immunogenic CD8+ T cell epitopes. Proc. Natl. Acad. Sci. U. S. A. 112, E1754-E1762 (2015)' were used. The non-immunogenic peptides in the dataset are ligand- eluted MHC-I presented self-peptides that have been antigenically processed and MHC-bound. They were filtered for a length of 9-11 amino acids, resulting in 4254 unique non-epitopes.

As all the peptides in the benchmark dataset are MHC binders, they could all be used as input for the workflow of Example 1 and the IEDB immunogenicity predictor. For Ineo-Epp, predictions were made for the nine HLA-I supertypes and only the scores for predicted binders were considered.

From the three tools' prediction scores, receiver operator characteristic (ROC) curves were constructed and are shown in **Figure 6****.** The curves show the true positive and the false positive rate of predictions for a decreasing cut-off score.

In the comparison of the three tools, the workflow according to Example 1 (41) outperformed the IEDB immunogenicity predictor (43) and Ineo-Epp (42). The Example 1 AUC was 0.84, which is close to the internal validation 0.88 AUC. The IEDB immunogenicity predictor had an AUC of 0.57. Ineo-Epp also had a lower performance (AUC of 0.65).

### Example 4: Cancer Vaccine

To demonstrate the validity of the immunogenicity scoring of the framework outlined in example 1, the framework is applied to data from a study published on a breast cancer vaccine.

The study, i.e. "Dillon, P. M. et al. A pilot study of the immunogenicity of a 9-peptide breast cancer vaccine plus poly-ICLC in early stage breast cancer. J. Immunother. Cancer 5, 1-10 (2017)", assessed the immune response of twelve patients with breast cancer to a vaccine consisting of nine MHC class I-restricted breast cancer-associated peptides. CD8+ T cell responses to the vaccine were assessed by both direct and stimulated interferon gamma ELISpot assays. Such ELISpot assay quantitatively assesses the frequency of cytokine secretion for a cell in response to a stimulant, and is therefore a suitable method to assess immunogenicity of said vaccine.

In the stimulated ELISpot assays, peptide specific CD8+ T-cell responses were detected in four out of eleven evaluable patients. Two ELISpot responses were observed in response to the modified HLA-A2 CEA571-579 peptide (YLS-D antigen) and two responses were observed in response to HLA-A3 CEA27-35 (HLF antigen). One patient had a borderline response to HLA-A3 MAGE-A196-104.

The framework of the present example was used to score the immunogenicity of each of the nine peptides in the vaccine (Table 1). As table 1 shows, the two antigens that resulted in positive ELISpot assays (YLS-D and HLF antigen) had the highest Framework/immunogenicity scores.

**Table 1 Vaccine peptides with ELISpot responses and Framework Score**

| Allele | Sequence | Epitope | ELISpot | Score |
|---|---|---|---|---|
| HLA-A1 | EADPTGHSY | MAGE-A1₁₆₁₋₁₆₉ | - | 0.11 |
| | EVDPIGHLY | MAGE-A3₁₆₈₋₁₇₆ | - | 0.13 |
| HLA-A2 | KIFGSLAFL | Her-2/neu₃₆₉₋₃₇₇ | - | 0.39 |
| | YLSGADLNL | CEA₅₇₁₋₅₇₉ | 2 responses | 0.49 |
| | GLYDGMEHL | MAGE-A1₀₂₅₄₋₂₆₂ | - | 0.30 |
| HLA-A3 | HLFGYSWYK | CEA₂₇₋₃₅ | 2 responses | 0.39 |
| | VLRENTSPK | Her-2/neu₇₅₄₋₇₆₂ | - | 0.25 |
| | SLFRAVITK | MAGGE-A1₉₆₋₁₀₄ | 1 borderline response | 0.28 |
| HLA-A3/ HLA-A31 | ASGPGGGAPR | NY-ESO-1₅₃₋₆₂ | - | 0.22 |

### Example 5: Virus Immunogenicity

Immunogenicity predictions of epitopes derived from viral proteins allow for rapid screening of new viruses on vaccination targets.

The immunogenicity classifier outlined in example 1 is trained on immunogenic peptides of already tested virus strains and can be used to pre-screen the full list of potential epitopes of a new virus strain. It reduces the number of in-vitro assays required for this particular strain, effectively minimising the associated timeframes and costs. Additionally, it aids in optimising the vaccine for target peptides that are likely to cause an immune response in a bigger portion of the population.

To show the applicability of the present algorithm for viral peptides, it was utilised to compare immunogenic epitopes of SARS-CoV-2 with epitopes of SARS-CoV and MERS-CoV. These three viruses are closely related yet elicit symptoms of varying orders of magnitude. Indeed, the symptoms of SARS-CoV are more severe (around 10% mortality) than for the current coronavirus (estimated at 1.3%), while MERS-CoV was even more deadly than both of them (20.4-69.2% mortality).

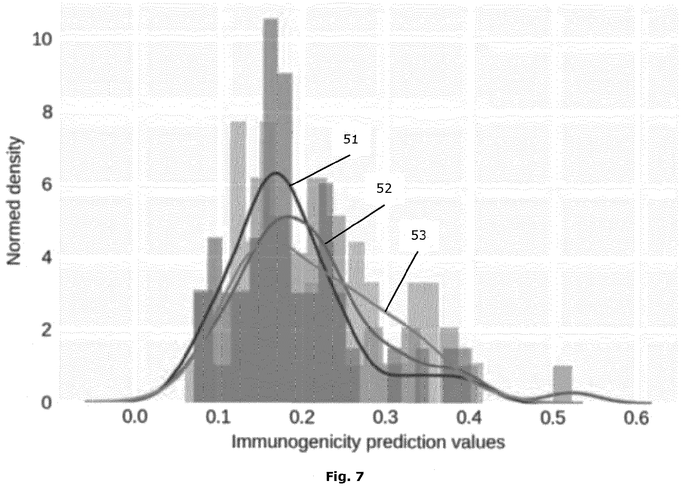

After gathering the sequences of the envelope proteins of SARS-CoV-2 as well as for SARS-CoV and MERS-CoV, all possible 9-mers were extracted. These were run through the neoMS algorithm to determine which ones are likely to be presented at the surface of infected cells. Subsequently, the algorithm of the present invention ranked the presented peptides according to their predicted immunogenicity. The resulting immunogenicity likelihood distribution of this reduced set of presented peptides was examined and, as can be seen in **Figure 7****,** SARS-CoV-2 exhibits on average fewer potentially immunogenic peptides than the SARS-CoV, which in turns has fewer likely immunogenic peptides than MERS-CoV. This implies that the immunogenic load of these three viruses does seem to correlate with their pathogenicity thereby corroborating the hypothesis by 'Mcmanus, L. M. & Mitchell, R. N. Pathobiology of human disease: a dynamic encyclopedia of dis-ease mechanisms. in Pathobiology of human disease: a dynamic encyclopedia of disease mechanisms. 1036 (Elsevier, 2014)'. **Figure 7** shows Histogram and associated density curves of immunogenicity probability for the envelope-derived cell-sur-face-presented peptides of SARS-CoV-2 (red), SARS-CoV (blue) and MERS-CoV (green).

### Example 6: Differential amino acid usage in peptides on which the positive and negative data set of the present invention are based

As discussed above, the positive and negative data sets are based on peptides either assumed to elicit an immune response (positive data set) or either assumed not to elicit an immune response (negative data set).

The differential amino acid usage analysis (dagLogo: https://doi.org/10.1371/ journal.pone.0242030) assessed whether there are conserved sequence patterns within the peptides of the positive and negative set and whether these patterns differ between the two groups. For this, the positive data set was based on immunogenic peptides with no match to the human proteome while the negative data set was based on MHC presented peptides with no match to the human proteome.

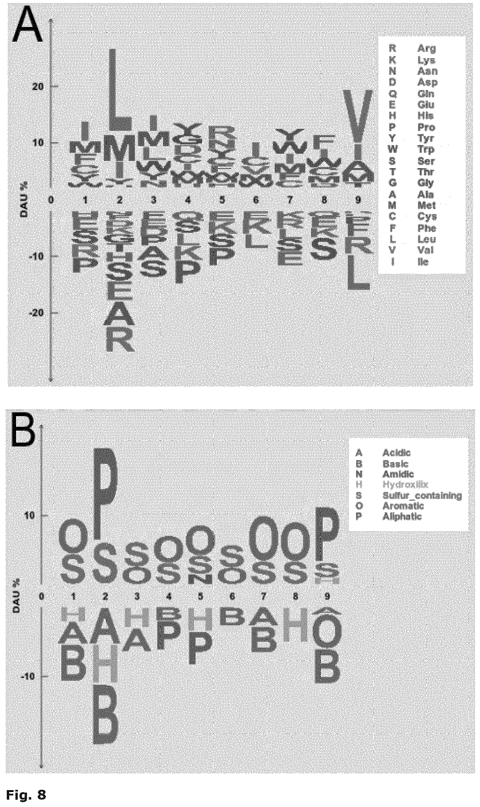

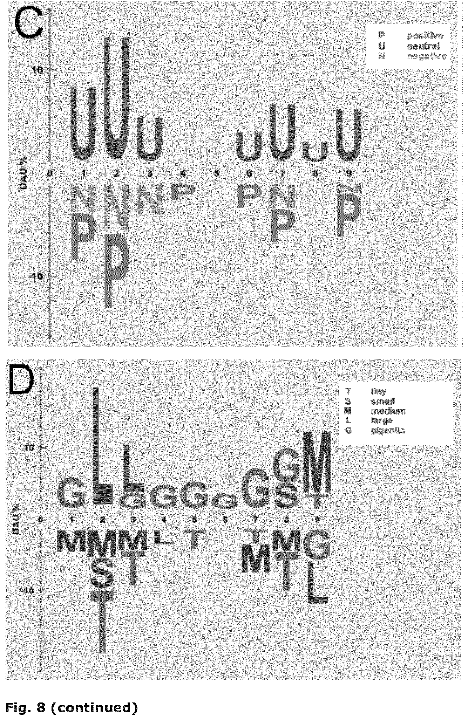

**Figure 8** shows peptide sequence logos showing differential amino acid usage of 9 amino acid long peptides in the immunogenic positive dataset (above) over 9 amino acid long peptides in the non-immunogenic negative dataset (below). Amino acids were either ungrouped (Fig. 8A), grouped by chemical properties (Fig. 8B, acidic, basic, amidic, hydroxylic, sulfur-containing, aromatic or aliphatic), electrical charge (Fig. 8C; positive, negative or neutral) or size (Fig. 8D; tiny, small, medium, large or gigantic).

From the figure, it is clear that there are physicochemical differences between both sets of peptides, on which the model is build and trained. Further, it also shows that certain amino acids or amino acid properties at certain positions are correlated with immunogenicity.

### Example 7: Assaying the efficiency of the computer-implemented method of the present invention on a newly generated dataset and comparing it with other available methods

An experimental immunogenicity testing of 33 peptides by ELISpot assay was performed on healthy donor T-cells. The assays were performed with two rounds of stimulation through autologous dendritic cells loaded with peptides. All tests were performed in duplicate or triplicate.

All peptides were predicted to be presented on at least two donor HLA alleles using netMHCpan4.0, a tool for prediction of binding of peptides to a MHC molecule using artificial neural networks. This tool is trained on naturally eluted ligands and on binding affinity data. It returns two properties: either the likelihood of a peptide becoming a natural ligand, or the predicted binding affinity.

A positive ELISpot result was defined as a minimum of 25 spots per 5×10⁴ cells and an increase of at least 2x compared to the control. Of the 33 peptides that were all predicted to be presented, eight peptides (23.5%) tested positive by ELISpot assay.

The same peptides were assayed for immunogenicity using the computer-implemented method of the present invention. The positive data set was based on immunogenic peptides with no match to the human proteome while the negative data set was based on MHC presented peptides with no match to the human proteome.

The different scoring systems:
- Scoring of the method of the present invention: using the computer-implemented method of the invention, an immunogenicity score was computed for each peptide.
- IEDB immunogenicity score: using the IEDB immunogenicity predictor, a score for each peptide was computed.
- netMHCpan rank score (HLA-A*02:01): using netMHCpan4.0, the netMHCpan rank score for each peptide for the HLA-A*02:01 allele was computed.
- netMHCpan rank score (all donor HLAs): using netMHCpan4.0, the netMHCpan rank score for each peptide was computed for all donor HLA alleles and the lowest score was considered.

**Figure 9A** shows the predicted likelihood of peptides being immunogenic as assayed using the computer-implemented method of the invention of pools of peptides with a positive or negative ELISpot result (p = 0.019). From those results, it is clear that peptides with a positive ELISpot result (n = 8) had a significantly higher immunogenicity score using the method of the present invention compared to peptides with a negative result (n = 25). The computer-implemented method of the present invention is therefore able to differentiate between immunogenic and non-immunogenic peptides (p=0.019). The difference was not significant for the IEDB and netMHCpan tools.

**Figure 9B** shows a comparison of receiver operating characteristics (ROC) curves of multiple different immunogenicity predictors, including the computer-implemented method of the invention, the IEDB immunogenicity predictor and netMHCpan4.0, the latter either based on a specific HLA allele or all HLA alleles of a subject. The area under the curve-values (AUC values) for the ROC curves were:

| **Predictor tool** | **AUC value** |
|---|---|
| Computer-implemented method of the invention | 0.80 |
| IEDB immunogenicity predictor | 0.47 |
| netMHCpan4.0 HLA-A02:01 | 0.52 |
| netMHCpan4.0 all donor HLAs | 0.42 |

The results show that the accuracy of differentiating between immunogenic and non-immunogenic peptides of the computer-implemented method of the present invention is better than the accuracy of the other methods, such as IEDB immunogenicity predictor and netMHCpan4.0, the latter only predicting binding affinity and not immunogenicity.

In a subsequent step, peptides were ordered from highest to lowest immunogenicity scores as obtained through the method of the present invention and presented in a lift curve. All positive peptides as assayed by ELISpot were present in the top 60% of the peptides, making it possible to discard 40% of all peptides without losing any true positive, as shown in **Figure 9C****,** which shows the percentage of true positives in the population (as cumulative gain). The cumulative gain curve illustrates what percentage of the true positives is identified with the smallest possible portion of samples. Compared to the IEDB immunogenicity predictor and netMHCpan4.0, using the method of the present invention results in a significant increase of specificity from 23% to 40% without losing sensitivity, substantially reducing the number of candidate neoantigens.

Concluding, the computer-implemented method of the present invention allows for ranking presented neoepitopes better by their immunogenic potential to improve neoepitope prediction and reduce false positives significantly. Prioritizing neoepitopes with high predicted immunogenicity by the computer-implemented method of the present invention increases the likelihood of selecting actionable neoantigens. The computer-implemented method of the present invention is thus a powerful tool both in improving target selection for personalized immunotherapies as well as in offering immunogenic peptide load as an improved biomarker for patient survival and response to therapy.

### Example 8: Identification of patients with low tumor mutational burden that would still benefit from immune checkpoint inhibitor therapy

A commonly used biomarker for selecting patients to receive immune checkpoint inhibitor therapy (ICI), a form of cancer therapy that aims to re-activate a patient's immune system, is a tumor mutational burden (TMB) of >100 (ns= nonsynonymous) mutations. A high TMB has been shown to be correlated with clinical benefit of ICI. However, there are some patients with low TMB, that would still benefit from ICI and it is crucial to identify those patients.

Three potential biomarkers for response to therapy were investigated in a cohort of low TMB melanoma patients receiving ICI.

The first biomarker was TMB (the overall number of non-synonymous mutations). The cohort was divided into two equally sized groups based on TMB, "High" containing the 50% of the patients with the highest number of mutations and "Low" containing the 50% of patients with the lowest number of mutations.

**Figure 10** shows a Kaplan Meier curve, which illustrates the probability of an event at a respective time interval. It can be seen that TMB **(****Fig.10** **left panel)** is not a good biomarker for identifying patients that show a response to therapy.

Subsequently, the experiment looked at the number of presented mutations per patient. The number of presented mutations was obtained by running netMHCpan4.1 for all possible peptides of 9-11 amino acid length resulting from each mutation. A mutation was considered presented if at least one peptide with a rank score < 2 was identified. The cohort was divided into two equally sized groups based on the number of presented mutations, "High" containing the 50% of the patients with the highest number of presented mutations and "Low" containing the 50% of patients with the lowest number of presented mutations.

**Fig. 10** **middle panel** shows that the number of presented mutations, similar to TMB, is not a good biomarker for identifying patients that show a response to therapy.

Finally, the experiment looked at the number of immunogenic mutations per patient. The number of immunogenic mutations was obtained by running netMHCpan4.1 for all possible peptides of 9-11 amino acid length resulting from each mutation. For all peptides considered to be presented (rank score < 2) an immunogenicity score was computed using the computer-implemented method of the present invention. A mutation was considered immunogenic if at least one peptide with an immunogenicity score > 0.75 was identified. The cohort was divided into two equally sized groups based on the number of immunogenic mutations, "High" containing the 50% of the patients with the highest number of immunogenic mutations and "Low" containing the 50% of patients with the lowest number of immunogenic mutations.

**Fig. 10** **right panel** shows that that the number of immunogenic mutations, is a better biomarker for identifying patients that show a response to therapy than TMB or the number of presented mutations.

This shows that it is not just the sheer number of mutations in a tumor that drive response to therapy, especially in patients with a low number of mutations, but also the immunogenic potential of these mutations. The computer-implemented method of the present invention therefore may therefore be used to identify patients with low tumor mutational burden that would still benefit from immune checkpoint inhibitor therapy, based on the immunogenic potential of the mutations.

The present invention is in no way limited to the embodiments described in the examples and/or shown in the figures. On the contrary, methods according to the present invention may be realized in many different ways without departing from the scope of the invention.

## Claims

1. Computer-implemented method for determining immunogenicity of a peptide, preferably a cell surface-presented peptide, comprising the steps of:
- obtaining an amino acid sequence of said peptide, wherein said peptide comprises proteinogenic and/or non-proteinogenic amino acids;
- obtaining for each proteinogenic amino acid a plurality of numeric indices, each related to a physicochemical property;
- obtaining a training data set comprising a positive data set and negative data set, wherein the positive set comprises (numerical) data related to a plurality of amino acid sequences of immunogenic peptides, wherein the negative set comprises (numerical) data related to a plurality of amino acid sequences of non-immunogenic peptides;
- training a mathematical classification model on the training data set;
- determining a likelihood of said peptide eliciting an immune response by means of the trained classification model;
wherein the method comprises the steps of:
- performing a principal component analysis on the numeric indices of each of the proteinogenic amino acids to obtain principal components for each analysed proteinogenic amino acid;
- obtaining a feature vector for the amino acid sequences of the training data set and the amino acid sequence of said peptide, wherein a feature vector for an amino acid sequence is obtained by replacing each amino acid of said amino acid sequence by one or more corresponding principal components;
wherein the classification model is trained on the feature vectors of the training data set;
wherein the likelihood of said peptide being immunogenic is determined for the feature vector of said peptide;
**characterized in that** the negative data set comprising (numerical) data related to a plurality of amino acid sequences of non-immunogenic peptides, is obtained by
- obtaining amino acid sequences of peptides capable of binding to and/or being presented on a MHC;
- obtaining amino acid sequences corresponding to proteome peptides;
- comparing the amino acid sequences of peptides capable of binding to and/or being presented on the MHC to a proteome amino acid sequence to determine matches therebetween;
the negative data set comprising the amino acid sequence of peptides capable of binding to and/or being present on the MHC that are closely related to but not identical to peptides of the proteome, said closely related amino acid sequences are having 1, 2 or 3 amino acid mismatches compared to peptides of the proteome.

2. Method according to preceding claim 1, wherein the negative data set comprising (numerical) data related to a plurality of amino acid sequences of non-immunogenic peptides, is obtained by:
- obtaining amino acid sequences of peptides capable of binding to and/or being presented on a major histocompatibility complex (MHC), preferably identified by means of binding assays and/or mass spectrometry;
- obtaining amino acid sequences corresponding to housekeeping proteins;
- comparing the amino acid sequences of peptides capable of binding to and/or being presented the MHC to the amino acid sequences corresponding to housekeeping proteins to determine matches therebetween;
the negative data set comprising the amino acid sequences of said matches.

3. Method according to preceding claim 1 or 2, wherein the obtained MHC presented amino acid sequences are linear sequences.

4. Method according to any of preceding claims 1 to 3, wherein the immunogenic peptides of the positive data set are obtained by:
- obtaining amino acid sequences of peptides capable of inducing a T-cell response;
- obtaining amino acid sequences corresponding to proteome peptides;
- comparing the T-cell response inducing amino acid sequences to the proteome amino acid sequences to determine matches therebetween;
the positive data set comprising the T-cell response inducing amino acid sequences except for the amino acid sequence of said matches.

5. Method according to any of the preceding claims 1 to 3, wherein the immunogenic peptides of the positive data set are obtained by:
- obtaining amino acid sequences of peptides capable of inducing a T-cell response;
- obtaining amino acid sequences corresponding to proteome peptides;
- comparing the T-cell response inducing amino acid sequences to proteome amino acid sequences to determine matches therebetween;
the positive data set comprising the T-cell response inducing amino acid sequences that are closely related to but not identical to peptides of the proteome, said closely related amino acid sequences are having 1, 2 or 3 amino acid mismatches compared to peptides of the proteome.

6. Method according to any of the preceding claim 4 or 5, wherein the T-cell response inducing amino acid sequences are linear sequences.

7. Method according to any of preceding claims, wherein the training data set comprises a positive and negative data set, wherein the negative data set comprises substantially more records compared to the positive data set.

8. Method according to any of preceding claims, wherein a feature vector for an amino acid sequence is obtained by replacing each amino acid of said amino acid sequence by at least 2 corresponding main principal components, preferably between 2 and 10 corresponding main principal components, most preferably 3 corresponding main principal components.

9. Method according to any of preceding claims, wherein prior to the principal component analysis the plurality numeric indices are transformed to z-values.

10. Method according to any of preceding claims, wherein the classification model is a supervised classification machine learning algorithm, more preferably wherein the machine learning algorithm is one or more of a Multilayer Perceptron classifier, a Gaussian Naive Bayes classifier, a Linear Support Vector Machine, a Kernel Support Vector Machine, a K-Nearest Neighbour classifier, or a Random Forest classifier, most preferably wherein the machine learning algorithm is a Random Forest classifier.

11. Computer system for determining immunogenicity of a peptide, the computer system being configured for performing the computer-implemented method according to any one of the preceding claims.

12. Computer program product for determining immunogenicity of a peptide, the computer program product comprising instructions which, when the computer program product is executed by a computer, cause the computer to carry out the computer-implemented method according to any one of the preceding claims.

13. Use of the computer-implemented method according to any of preceding claims 1 to 10 and/or the computer system according to preceding claim 11 and/or the computer program product according to preceding claim 12, for determining a cancer treatment of a subject, preferably by determining immunogenicity of a neoantigen presented by a tumour cell of the subject.

14. Use of the computer-implemented method according to any of preceding claims 1 to 10 and/or the computer system according to preceding claim 11 and/or the computer program product according to preceding claim 12, for screening of a virus or bacterium on a vaccination target, preferably by determining immunogenicity of epitopes from viral or bacterial proteins.

15. Use of the computer-implemented method according to any of preceding claims 1 to 10 and/or the computer system according to preceding claim 11 and/or the computer program product according to preceding claim 12, for characterizing autoimmunity reactions of a subject, preferably by determining immunogenicity of self-antigens presented by a cell of the subject.

16. Use of the computer-implemented method according to any of preceding claims 1 to 10 and/or the computer system according to preceding claim 11 and/or the computer program product according to preceding claim 12, for assessing immunogenicity changes of a peptide upon altering one or more amino acids of the amino acid sequence of said peptide.

## Patentansprüche

1. Computerimplementiertes Verfahren zum Bestimmen der Immunogenität eines Peptids, vorzugsweise eines an einer Zelloberfläche präsentierten Peptids, folgende Schritte umfassend:
- Gewinnen einer Aminosäuresequenz des Peptids, wobei das Peptid proteinogene und/oder nicht-proteinogene Aminosäuren umfasst,
- Gewinnen mehrerer numerischer Indizes für jede proteinogene Aminosäure, die jeweils mit einer physikochemischen Eigenschaft in Beziehung stehen,
- Gewinnen eines Trainingsdatensatzes, der einen positiven Datensatz und einen negativen Datensatz umfasst, wobei der positive Satz (numerische) Daten umfasst, die mit mehreren Aminosäuresequenzen von immunogenen Peptiden in Beziehung stehen, wobei der negative Satz (numerische) Daten umfasst, die mit mehreren Aminosäuresequenzen von nicht-immunogenen Peptiden in Beziehung stehen,
- Trainieren eines Modells zur mathematischen Klassifizierung anhand des Trainingsdatensatzes,
- Bestimmen einer Wahrscheinlichkeit, dass das Peptid eine Immunreaktion hervorruft, mittels des trainierten Klassifizierungsmodells,
wobei das Verfahren die folgenden Schritte umfasst:
- Durchführen einer Hauptkomponentenanalyse an den numerischen Indizes jeder der proteinogenen Aminosäuren, um Hauptkomponenten für jede analysierte proteinogene Aminosäure zu gewinnen,
- Gewinnen eines Merkmalsvektors für die Aminosäuresequenzen des Trainingsdatensatzes und die Aminosäuresequenz des Peptids, wobei ein Merkmalsvektor für eine Aminosäuresequenz durch Ersetzen jeder Aminosäure der Aminosäuresequenz durch eine oder mehrere entsprechende Hauptkomponenten gewonnen wird,
wobei das Klassifizierungsmodell anhand der Merkmalsvektoren des Trainingsdatensatzes trainiert wird,
wobei die Wahrscheinlichkeit, dass das Peptid immunogen ist, für den Merkmalsvektor des Peptids bestimmt wird,
**dadurch gekennzeichnet, dass** der negative Datensatz, der (numerische) Daten umfasst, welche mit mehreren Aminosäuresequenzen von nicht-immunogenen Peptiden in Beziehung stehen, durch Folgendes gewonnen wird:
- Gewinnen von Aminosäuresequenzen von Peptiden, die in der Lage sind, an einen MHC zu binden und/oder darauf präsentiert zu werden,
- Gewinnen von Aminosäuresequenzen, die Proteompeptiden entsprechen,
- Vergleichen der Aminosäuresequenzen von Peptiden, die in der Lage sind, an den MHC zu binden und/oder darauf präsentiert zu werden, mit einer Proteom-Aminosäuresequenz, um Übereinstimmungen zwischen diesen zu bestimmen,
wobei der negative Datensatz die Aminosäuresequenz von Peptiden umfasst, die in der Lage sind, an den MHC zu binden und/oder darauf präsentiert zu werden, die in enger Beziehung zu Peptiden des Proteoms stehen, jedoch nicht mit diesen identisch sind, wobei die in enger Beziehung stehenden Aminosäuresequenzen im Vergleich zu Peptiden des Proteoms 1, 2 oder 3 Aminosäure-Nichtübereinstimmungen aufweisen.

2. Verfahren nach dem vorhergehenden Anspruch 1, wobei der negative Datensatz, der (numerische) Daten umfasst, welche mit den mehreren Aminosäuresequenzen von nicht-immunogenen Peptiden in Beziehung stehen, durch Folgendes gewonnen wird:
- Gewinnen von Aminosäuresequenzen von Peptiden, die in der Lage sind, an einen Haupthistokompatibilitätskomplex (MHC) zu binden und/oder darauf präsentiert zu werden, vorzugsweise mittels Bindungs-Assay und/oder Massenspektrometrie identifiziert,
- Gewinnen von Aminosäuresequenzen, die Housekeeping-Proteinen entsprechen,
- Vergleichen der Aminosäuresequenzen der Peptide, die in der Lage sind, an den MHC zu binden und/oder darauf präsentiert zu werden, mit den Aminosäuresequenzen, die Housekeeping-Proteinen entsprechen, um Übereinstimmungen zwischen diesen zu bestimmen,
wobei der negative Datensatz die Aminosäuresequenzen dieser Übereinstimmungen umfasst.

3. Verfahren nach dem vorhergehenden Anspruch 1 oder 2, wobei die gewonnenen an dem MHC präsentierten Aminosäuresequenzen lineare Sequenzen sind.

4. Verfahren nach einem der vorhergehenden Ansprüche 1 bis 3, wobei die immunogenen Peptide des positiven Datensatzes durch Folgendes gewonnen werden:
- Gewinnen von Aminosäuresequenzen von Peptiden, die in der Lage sind, eine T-Zellenreaktion zu induzieren,
- Gewinnen von Aminosäuresequenzen, die Proteompeptiden entsprechen,
- Vergleichen der eine T-Zellenreaktion induzierenden Aminosäuresequenzen mit den Proteom-Aminosäuresequenzen, um Übereinstimmungen zwischen diesen zu bestimmen,
wobei der positive Datensatz, die eine T-Zellenreaktion induzierenden Aminosäuresequenzen mit Ausnahme der Aminosäuresequenz der Übereinstimmungen umfasst.

5. Verfahren nach einem der vorhergehenden Ansprüche 1 bis 3, wobei die immunogenen Peptide des positiven Datensatzes durch Folgendes gewonnen werden:
- Gewinnen von Aminosäuresequenzen von Peptiden, die in der Lage sind, eine T-Zellenreaktion zu induzieren,
- Gewinnen von Aminosäuresequenzen, die Proteompeptiden entsprechen,
- Vergleichen der eine T-Zellenreaktion induzierenden Aminosäuresequenzen mit Proteom-Aminosäuresequenzen, um Übereinstimmungen zwischen diesen zu bestimmen,
wobei der positive Datensatz die eine T-Zellenreaktion induzierenden Aminosäuresequenzen umfasst, die in enger Beziehung mit den Peptiden des Proteoms stehen, jedoch nicht mit diesen identisch sind, wobei die in enger Beziehung stehenden Aminosäuresequenzen im Vergleich zu Peptiden des Proteoms 1, 2 oder 3 Aminosäure-Nichtübereinstimmungen aufweisen.

6. Verfahren nach einem der vorhergehenden Ansprüche 4 oder 5, wobei die eine T-Zellenreaktion induzierenden Aminosäuresequenzen lineare Sequenzen sind.

7. Verfahren nach einem der vorhergehenden Ansprüche, wobei der Trainingsdatensatz einen positiven und einen negativen Datensatz umfasst, wobei der negative Datensatz im Wesentlichen mehr Aufzeichnungen als der positive Datensatz umfasst.

8. Verfahren nach einem der vorhergehenden Ansprüche, wobei ein Merkmalsvektor für eine Aminosäuresequenz durch Ersetzen jeder Aminosäure der Aminosäuresequenz durch mindestens zwei entsprechende Hauptkomponenten gewonnen wird, vorzugsweise zwischen 2 und 10 entsprechende Hauptkomponenten, am meisten bevorzugt 3 entsprechende Hauptkomponenten.

9. Verfahren nach einem der vorhergehenden Ansprüche, wobei die mehreren numerischen Indizes vor der Hauptkomponentenanalyse in Z-Werte umgewandelt werden.

10. Verfahren nach einem der vorhergehenden Ansprüche, wobei das Klassifizierungsmodell ein Klassifizierungsalgorithmus für überwachtes maschinelles Lernens ist, wobei der maschinelle Lernalgorithmus bevorzugter einer oder mehrere von einem Mehrlagiges-Perzeptron-Klassifikator, einem Gaußschen naiven Bayes-Klassifikator, einer Linear-Support-Vector-Machine, einer Kernel-Support-Vector-Machine, einem K-Nächste-Nachbarn-Klassifikator oder einem Random-Forest-Klassifikator ist, wobei der maschinelle Lernalgorithmus am meisten bevorzugt ein Random-Forest-Klassifikator ist.

11. Computersystem zum Bestimmen der Immunogenität eines Peptids, wobei das Computersystem dafür konfiguriert ist, das computerimplementierte Verfahren nach einem der vorhergehenden Ansprüche durchzuführen.

12. Computerprogrammprodukt zum Bestimmen der Immunogenität eines Peptids, wobei das Computerprogrammprodukt Anweisungen umfasst, die bei Ausführung des Computerprogrammprodukts durch einen Computer den Computer veranlassen, das computerimplementierte Verfahren nach einem der vorhergehenden Ansprüche auszuführen.

13. Verwendung des computerimplementierten Verfahrens nach einem der vorhergehenden Ansprüche 1 bis 10 und/oder des Computersystems nach dem vorhergehenden Anspruch 11 und/oder des Computerprogrammprodukts nach dem vorhergehenden Anspruch 12 zum Bestimmen einer Krebsbehandlung eines Subjekts, vorzugsweise durch Bestimmen der Immunogenität eines Neoantigens, das durch eine Tumorzelle des Subjekts präsentiert wird.

14. Verwendung des computerimplementierten Verfahrens nach einem der vorhergehenden Ansprüche 1 bis 10 und/oder des Computersystems nach dem vorhergehenden Anspruch 11 und/oder des Computerprogrammprodukts nach dem vorhergehenden Anspruch 12 zum Screenen eines Virus oder Bakteriums auf ein Impfstoffziel, vorzugsweise durch Bestimmen der Immunogenität von Epitopen von viralen oder bakteriellen Proteinen.

15. Verwendung des computerimplementierten Verfahrens nach einem der vorhergehenden Ansprüche 1 bis 10 und/oder des Computersystems nach dem vorhergehenden Anspruch 11 und/oder des Computerprogrammprodukts nach dem vorhergehenden Anspruch 12 zum Charakterisieren von Autoimmunreaktionen eines Subjekts, vorzugsweise durch Bestimmen der Immunogenität von Selbstantigenen, die durch eine Zelle des Subjekts präsentiert werden.

16. Verwendung des computerimplementierten Verfahrens nach einem der vorhergehenden Ansprüche 1 bis 10 und/oder des Computersystems nach dem vorhergehenden Anspruch 11 und/oder des Computerprogrammprodukts nach dem vorhergehenden Anspruch 12 zum Bewerten von Änderungen der Immunogenität eines Peptids nach dem Modifizieren einer oder mehreren Aminosäuren der Aminosäuresequenz des Peptids.

## Revendications

1. Procédé mis en oeuvre par ordinateur pour déterminer l'immunogénicité d'un peptide, de préférence un peptide présenté à la surface d'une cellule, comprenant les étapes consistant à:
- obtenir une séquence d'acides aminés dudit peptide, dans lequel ledit peptide comprend des acides aminés protéinogènes et/ou non protéinogènes;
- obtenir pour chaque acide aminé protéinogène une pluralité d'indices numériques, chacun lié à une propriété physico-chimique;
- obtenir un ensemble de données d'entraînement comprenant un ensemble de données positif et un ensemble de données négatif, dans lequel l'ensemble positif comprend des données (numériques) liées à une pluralité de séquences d'acides aminés de peptides immunogènes, dans lequel l'ensemble négatif comprend des données (numériques) liées à une pluralité de séquences d'acides aminés de peptides non immunogènes;
- entraîner un modèle de classification mathématique sur l'ensemble de données d'entraînement;
- déterminer la probabilité que ledit peptide suscite une réponse immunitaire au moyen du modèle de classification entraîné;
dans lequel le procédé comprend les étapes consistant à:
- effectuer une analyse en composantes principales sur les indices numériques de chacun des acides aminés protéinogènes pour obtenir des composantes principales pour chaque acide aminé protéinogène analysé;
- obtenir un vecteur de caractéristiques pour les séquences d'acides aminés de l'ensemble de données d'entraînement et la séquence d'acides aminés dudit peptide, dans lequel un vecteur de caractéristiques pour une séquence d'acides aminés est obtenu en remplaçant chaque acide aminé de ladite séquence d'acides aminés par une ou plusieurs composantes principales correspondants;
dans lequel le modèle de classification est entraîné sur les vecteurs de caractéristiques de l'ensemble de données d'entraînement;
dans lequel la probabilité que ledit peptide soit immunogène est déterminée pour le vecteur de caractéristiques dudit peptide;
**caractérisé en ce que** l'ensemble de données négatif comprenant des données (numériques) liées à une pluralité de séquences d'acides aminés de peptides non immunogènes, est obtenu par
- l'obtention de séquences d'acides aminés de peptides capables de se lier à et/ou d'être présentés sur un CMH;
- l'obtention de séquences d'acides aminés correspondant à des peptides du protéome;
- la comparaison des séquences d'acides aminés de peptides capables de se lier et/ou d'être présentés sur le CMH à une séquence d'acides aminés du protéome pour déterminer des correspondances entre elles;
l'ensemble de données négatif comprenant la séquence d'acides aminés de peptides capables de se lier et/ou d'être présents sur le CMH qui sont étroitement liés mais non identiques aux peptides du protéome, lesdites séquences d'acides aminés étroitement liées présentant 1, 2 ou 3 mésappariements d'acides aminés par rapport aux peptides du protéome.

2. Procédé selon la revendication précédente 1, dans lequel l'ensemble de données négatif comprenant des données (numériques) liées à une pluralité de séquences d'acides aminés de peptides non immunogènes, est obtenu par:
- l'obtention de séquences d'acides aminés de peptides capables de se lier à et/ou d'être présentés sur un complexe majeur d'histocompatibilité (CMH), de préférence identifiés au moyen d'analyses de liaison et/ou d'une spectrométrie de masse;
- l'obtention de séquences d'acides aminés correspondant à des protéines de ménage;
- la comparaison des séquences d'acides aminés de peptides capables de se lier et/ou d'être présentés sur le CMH aux séquences d'acides aminés correspondant à des protéines de ménage pour déterminer des correspondances entre elles;
l'ensemble de données négatif comprenant les séquences d'acides aminés desdites correspondances.

3. Procédé selon la revendication précédente 1 ou 2, dans lequel les séquences d'acides aminés présentés sur le CMH obtenues sont des séquences linéaires.

4. Procédé selon l'une quelconque des revendications précédentes 1 à 3, dans lequel les peptides immunogènes de l'ensemble de données positif sont obtenus par:
- l'obtention de séquences d'acides aminés de peptides capables d'induire une réponse des lymphocytes T;
- l'obtention de séquences d'acides aminés correspondant à des peptides du protéome;
- la comparaison des séquences d'acides aminés induisant une réponse des lymphocytes T aux séquences d'acides aminés du protéome pour déterminer des correspondances entre elles;
l'ensemble de données positif comprenant les séquences d'acides aminés induisant la réponse des lymphocytes T, à l'exception de la séquence d'acides aminés desdites correspondances.

5. Procédé selon l'une quelconque des revendications précédentes 1 à 3, dans lequel les peptides immunogènes de l'ensemble de données positif sont obtenus par:
- l'obtention de séquences d'acides aminés de peptides capables d'induire une réponse des lymphocytes T;
- l'obtention de séquences d'acides aminés correspondant à des peptides du protéome;
- la comparaison des séquences d'acides aminés induisant une réponse des lymphocytes T à des séquences d'acides aminés du protéome pour déterminer des correspondances entre elles;
l'ensemble de données positif comprenant les séquences d'acides aminés induisant une réponse des lymphocytes T qui sont étroitement liées mais non identiques aux peptides du protéome, lesdites séquences d'acides aminés étroitement liées présentant 1, 2 ou 3 mésappariements d'acides aminés par rapport aux peptides du protéome.

6. Procédé selon l'une quelconque des revendications précédentes 4 ou 5, dans lequel les séquences d'acides aminés induisant une réponse des lymphocytes T sont des séquences linéaires.

7. Procédé selon l'une quelconque des revendications précédentes, dans lequel l'ensemble de données d'entraînement comprend un ensemble de données positif et négatif, dans lequel l'ensemble de données négatif comprend sensiblement plus d'enregistrements par rapport à l'ensemble de données positif.

8. Procédé selon l'une quelconque des revendications précédentes, dans lequel un vecteur de caractéristiques pour une séquence d'acides aminés est obtenu en remplaçant chaque acide aminé de ladite séquence d'acides aminés par au moins 2 composantes principales correspondantes, de préférence entre 2 et 10 composantes principales correspondantes, le plus préférablement 3 composantes principales correspondantes.

9. Procédé selon l'une quelconque des revendications précédentes, dans lequel avant l'analyse en composantes principales, la pluralité d'indices numériques est transformée en valeurs z.

10. Procédé selon l'une quelconque des revendications précédentes, dans lequel le modèle de classification est un algorithme d'apprentissage automatique de classification supervisée, plus préférentiellement, dans lequel l'algorithme d'apprentissage automatique est un ou plusieurs parmi un classificateur Perceptron multicouche, un classificateur bayésien naïf gaussien, une machine à vecteurs de support linéaire, une machine à vecteurs de support de noyau, un classificateur des K plus proches voisins, ou un classificateur de forêt aléatoire, idéalement dans lequel l'algorithme d'apprentissage automatique est un classificateur de forêt aléatoire.

11. Système informatique pour déterminer l'immunogénicité d'un peptide, le système informatique étant configuré pour mettre en oeuvre le procédé mis en oeuvre par ordinateur selon l'une quelconque des revendications précédentes.

12. Produit programme informatique pour déterminer l'immunogénicité d'un peptide, le produit programme informatique comprenant des instructions qui, lorsque le produit programme informatique est exécuté par un ordinateur, amènent l'ordinateur à exécuter le procédé mis en oeuvre par ordinateur selon l'une quelconque des revendications précédentes.

13. Utilisation du procédé mis en oeuvre par ordinateur selon l'une quelconque des revendications précédentes 1 à 10 et/ou du système informatique selon la revendication précédente 11 et/ou du produit programme informatique selon la revendication précédente 12, pour déterminer un traitement contre le cancer d'un sujet, de préférence en déterminant l'immunogénicité d'un néoantigène présenté par une cellule tumorale du sujet.

14. Utilisation du procédé mis en oeuvre par ordinateur selon l'une quelconque des revendications précédentes 1 à 10 et/ou du système informatique selon la revendication précédente 11 et/ou du produit programme informatique selon la revendication précédente 12, pour le dépistage d'un virus ou d'une bactérie sur une cible de vaccination, de préférence en déterminant l'immunogénicité des épitopes de protéines virales ou bactériennes.

15. Utilisation du procédé mis en oeuvre par ordinateur selon l'une quelconque des revendications précédentes 1 à 10 et/ou du système informatique selon la revendication précédente 11 et/ou du produit programme informatique selon la revendication précédente 12, pour caractériser des réactions d'auto-immunité d'un sujet, de préférence en déterminant l'immunogénicité d'auto-antigènes présentés par une cellule du sujet.

16. Utilisation du procédé mis en oeuvre par ordinateur selon l'une quelconque des revendications précédentes 1 à 10 et/ou du système informatique selon la revendication précédente 11 et/ou du produit programme informatique selon la revendication précédente 12, pour évaluer des changements d'immunogénicité d'un peptide lors de la modification d'un ou plusieurs acides aminés de la séquence d'acides aminés dudit peptide.
